# EUROPEAN PATENT APPLICATION

(11) **EP 4 778 532 A2**
(43) Date of publication of application: **22.07.2026**
(21) Application number: 26182945.1
(22) Date of filing: 29.11.2019
(51) Int. Cl.: A61K 36/064

(54) **METHODS FOR GROWING THE POPULATION OF MICROORGANISMS IN A GUT OF MONOGASTRIC ANIMALS**

(30) Priority: 14.06.2019 EP 19305767
(62) Divisional of application: 19812771.4
(71) Applicant: DANSTAR FERMENT AG, 6300 Zug (CH)
(72) Inventor: APPER, Emmanuelle, 31702 Blagnac (FR); CASTEX, Mathieu, 31702 Blagnac (FR); ACHARD, Caroline, 31702 Blagnac (FR)
(74) Representative: J A Kemp LLP

(57) **Abstract**

The present invention relates to a composition for growing the population of microorganisms in a gut of monogastric animals, and methods for growing the same. The present invention also relates to a method for enhancing an average daily gain of monogastric animals.

## Description

### FIELD

The present invention relates to a composition for growing the population of microorganisms in a gut of monogastric animals and methods for growing the same. The present invention also relates to a method for enhancing an average daily gain of monogastric animals.

### BACKGROUND

It is known that healthy, disease-free animals grow faster or are more able to convert their feed efficiently into body tissue than sick or immune-challenged animals. Obviously, faster growth or a more efficient conversion of feed into desirable body tissue in an animal is both economically and ecologically important, especially in animals raised for food. For this, and other reasons, it is desirable to prevent animals from contacting diseases.

One approach to keeping animals healthy is to give the animals antibiotics. However, that approach is not desirable for animals raised for food because there can be antibiotic residues in the food. Furthermore, using antibiotics increases the risk to select antibioresistant bacteria, which is a human health concern of crucial importance.

In another approach, live yeast supplementation to ruminants has been shown to enhancing fiber digestion and improve the growth thereof. A number of in vivo experiments have demonstrated in ruminants the effect of live yeast in enhancing fiber digestion (Wohlt et al., 1988; Guedes et al., 2008; Marden et al., 2008), and have concluded that one of the main mechanisms by which this is achieved is by increasing the growth and activities of fibrolytic bacteria community, including *Fibrobacteres succinogenes* (Chaucheyras-Durand and Fonty, 2002; Mosoniet al., 2007; Wallace and Newbold, 2007). *Fibrobacteres* is a bacterial phylum first described in ruminants. Only the genus *Fibrobacter* has been described for this phylum, and this genus presently contains only formally cultured and described species, *Fibrobacter intestinalis* and *Fibrobacter succinogenes. Fibrobacteres* is known to possess a unique array of hemicellulose-degrading enzymes and is an efficient and prolific degrader of cellulose as its sole energy source (Suen et al., 2011).

The knowledge about *Fibrobacteres* in monogastrics, and more particurlarly in swine is however less advanced. Culture-dependent approaches using selective medium and culture-independent techniques targeting 16S rDNA identified a potential core population inhabiting the swine gut population, including the genera *Fibrobacter. Fibrobacter intestinalis* and *Fibrobacter succinogenes* have been both identified in the gut of swine. Very little is known about interactions between probiotics and *Fibrobacteres* in monogastric animals. The only published result is a study about supplementation of post-weaned piglets for 28 days post-weaning with a probiotic bacteria leads to a decrease of *Fibrobacteres* population (Li et al., 2016). Contrary to what it is known and observed in ruminants, the effects of yeast on the activity and the growth of fibrolytic community, and more precisely of *Fibrobacteres,* in monogastric animals are not documented.

Another low studied phylum is the phylum of *Actinobacteria,* a phylum considered as minor in terms of relative abundance, but for which importance of the functionality is more and more claimed. The family of *Bifidobacteriaceae,* an important fibrolytic family is part of the *Actinobacteria.* Similarly, the family of *Coriobacteraceae* belongs to the phylum of *Actinobacteria.* Several bacteria that are part of this family are involved in the biliary acids metabolism, an interesting target to promote feed efficiency and growth performance of animals.

Fiber degradation is important for monogastric animal, such as, for example swine, as among other benefits it produces short-chain fatty acids (SCFA), an energy source for the colonocytes.

In the same manner, conversion of primary biliary acids into secondary biliary acids to improve growth performance (Ipharaguerre et al., 2018) is also growing in importance. Modulating gut microbiota to promote balance between primary and secondary biliary acids is thus interesting to optimise feed utilization and growth performance of monogastric animals.

The phyla *Fibrobacteres* and *Actinobacteria* are minor bacterial phyla in terms of relative abundance, however, they can have a really important functional role due to the fact that they exert fibrolytic effects and that the phylum of *Actinobacteria* contain bacteria able to convert bile acids (for example *Collinsella sp.* and *Olsenella sp.*)*.* In the gut, *Coriobacteriaceae* carry out functions of importance such as the conversion of bile salts and steroids as well as the activation of dietary polyphenols (Clavel et al., 2014). *Coriobacteriaceae* is a family within the order *Coriobacteriales* (phylum *Actinobacteria*)*.*

There is therefore a need for maintaining or growing the population of microorganisms responsible of fiber digestion, conversion of bile acids, or both in a gut of monogastric animals and/or a litter thereof to enhance fiber digestion of the monogastric animals. There is also an increased need for enhancing average daily gain of monogastric animals.

### SUMMARY

The present disclosure provides a composition comprising at least one biologically pure culture of *Saccharomyces cerevisiae* strain in amount effective for enhancing an average daily gain of monogastric animals, and a suitable carrier.

The present disclosure also provides composition comprising at least one biologically pure culture of *Saccharomyces cerevisiae* strain in amount effective for maintaining or growing a population of microorganisms in a gut of monogastric animals and/or a litter thereof, and a suitable carrier. In an aspect of the present disclosure, the population of microorganisms is responsible of fiber digestion, of conversion of primary biliary acids into secondary biliary acids or both.

The present invention further provides a feed or food additive comprising the composition as defined in the embodiments of the present disclosure.

The present disclosure also provide a use of the composition as defined in the embodiments of the present disclosure, for enhancing an average daily gain of monogastric animals.

The present disclosure also provide a method of maintaining or growing the population of microorganisms in a gut of monogastric animals and/or a litter thereof, the method comprising feeding to the monogastric animals an effective amount of the composition as defined in the embodiments of the present disclosure.

The present disclosure also provide a method for enhancing an average daily gain of monogastric animals, the method comprising feeding to the monogastric animals an effective amount of the composition as defined in the embodiments of the present disclosure.

### DESCRIPTION OF THE FIGURES

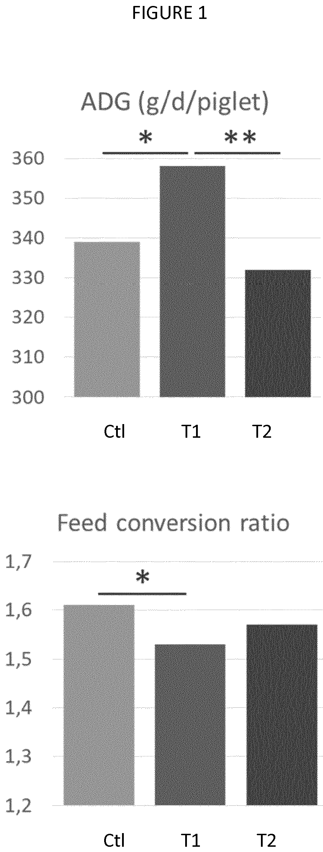

Figure 1 shows the Average Daily Gain (ADG) and feed conversion ratio obtained in piglets fed antibiotics alone (Control (Ctl)); *Saccharomyces cerevisiae var. boulardii* CNCM I-1079 combined with antibiotics (T1), and *Saccharomyces cerevisiae var. boulardii* CNCM I-1079 without antibiotics (T2) during the entire trial.

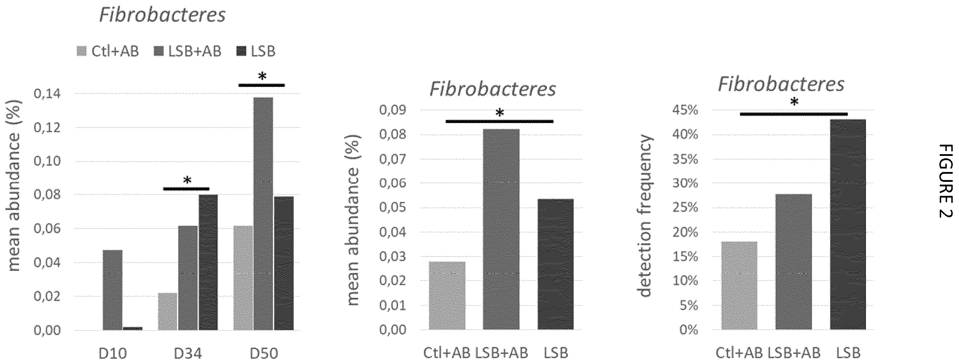

Figure 2 shows the mean abundance and detection frequency of *Fibrobacteres* phylum in piglets fed with antibiotics alone (Control (Ctl+AB)); *Saccharomyces cerevisiae var. boulardii* CNCM I-1079 combined with antibiotics (LSB+AB), and *Saccharomyces cerevisiae var. boulardii* CNCM I-1079 without antibiotics (LSB) at different time (Day (D) 10, 34 and 50) and on average. The first plot (on the left) shows the results per time points (Day 10, 34 and 50). The second plot (in the middle) shows the same result expressed on average per treatment during the whole experiment and the last figure (on the right) shows the average detection frequency per treatment.

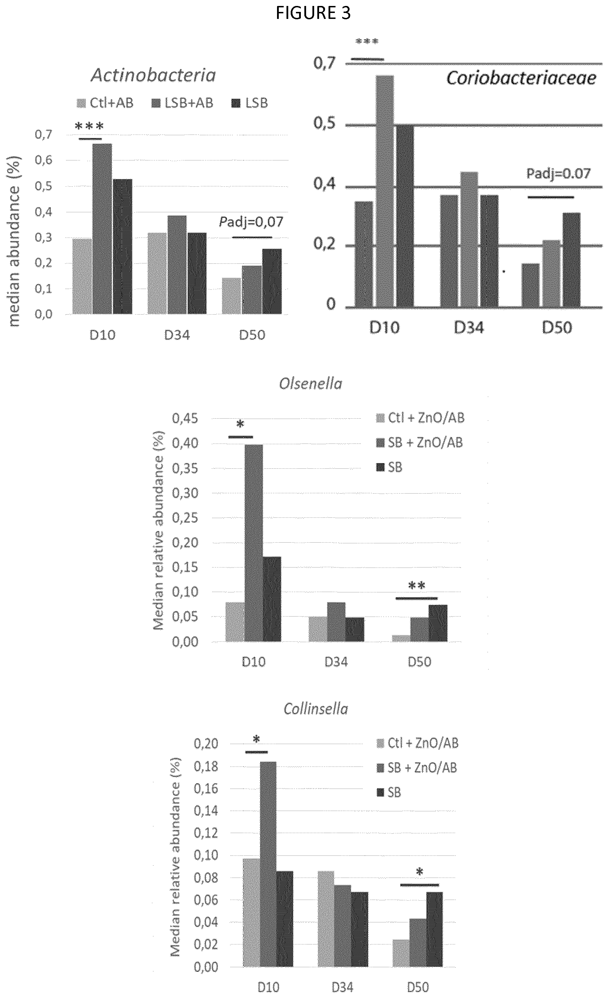

Figure 3 shows the median abundance of *Actinobacteria* phylum, *Coriobacteriaceae* family, and *Collinsella sp. and Olsenella sp.* in piglets fed with antibiotics alone (Ctl+AB ); *Saccharomyces cerevisiae var. boulardii* CNCM I-1079 + Antibiotitics (LSB+AB ), and *Saccharomyces cerevisiae var. boulardii* CNCM I-1079 alone (LSB).

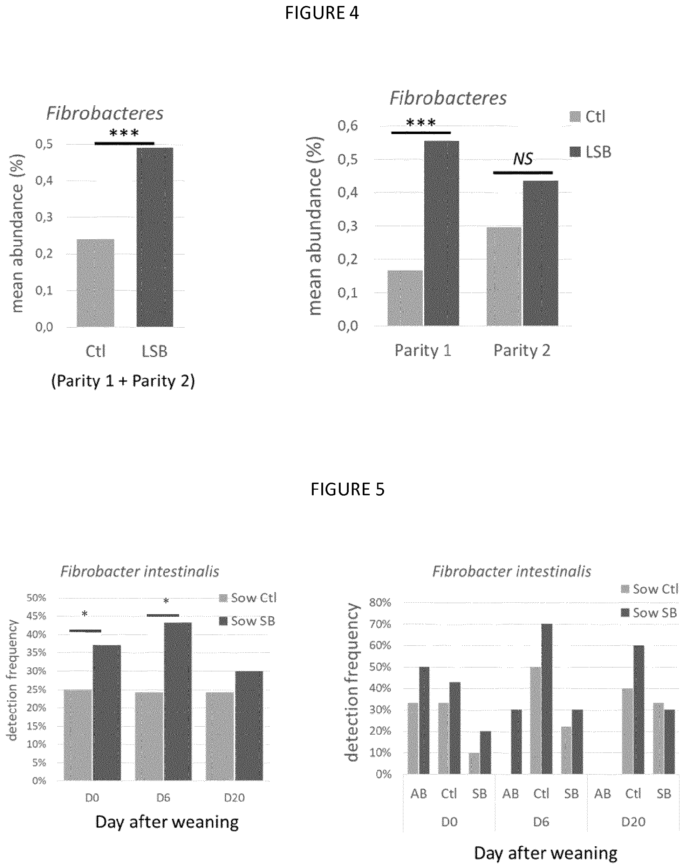

Figure 4 shows the mean abundance of *Fibrobacteres* in sows fed with Control diet (Ctl) or with *Saccharomyces cerevisiae var. boulardii* CNCM I-1079 (LSB), on average (left-hand panel) and per parity (right hand panel).
Figure 5 - On the left: Detection frequency of *Fibrobacter intestinalis* in piglets from mothers fed either with Control diet (Ctl) or with *Saccharomyces cerevisiae var. boulardii* CNCM I-1079 (LSB). On the right: effect of the treatment received by the sow (Ctl or LSB) and by the piglets (AB: Positive control, with antibiotic; Ctl: Negative control, without antibiotic and SB: *Saccharomyces cerevisiae var. boulardii* CNCM I-1079 diet) on the detection frequency of *F. intestinalis* per time points (day 0, 6, and 20). The antibiotic given to the piglets results in no detection of *F. intestinalis* in piglet feces, except when the sow was fed with *Saccharomyces cerevisiae var. boulardii* CNCM I-1079. When piglets received the negative control (Ctl), the supplementation of the sows with *Saccharomyces cerevisiae var. boulardii* CNCM I-1079 results in an increase of the detection frequency of *F. intestinalis* in piglet feces.

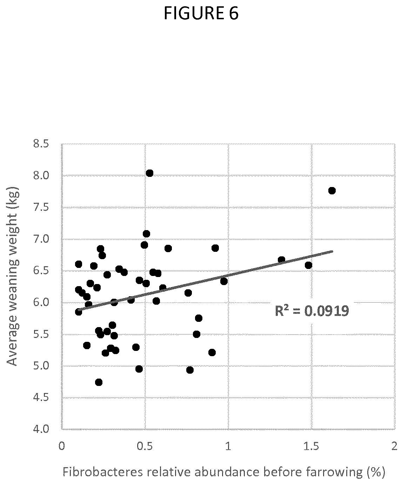

Figure 6 shows positive correlation of Fibrobacteres relative abundance in sows before farrowing (%) and average weaning weight of piglets (kg).

### DESCRIPTION

Most nutrients from the feed are chemically digested and absorbed by the small intestine. This is the case, in general, for protein, lipids, and digestible carbohydrates. However, a big portion of the non-digestible carbohydrates will reach the hindgut where they will be partially consumed by the local microbial communities, the microbiota. Short Chain Fatty Acids (SCFAs) are formed as a product of this microbial fermentation, and can be absorbed locally and used as a source of energy for the host. The fiber fraction of the feed is very heterogeneous and may include:
Soluble components, which are easily fermented such as, for example, fructans, gums and/or pectins;
Partially degradable structural components such as cellulose and/or hemicellulose;
Cell wall protecting substances, which are practically indigestible, like chitin and/or lignin.

The inclusion of fiber in swine diets stimulates the speed of the digestive transit in relation with its content in Neutral Detergent Fiber (NDF) and benefits animal welfare, reducing constipation incidence, stereotypic behaviors and stress (Gerrits and Verstegen, 2006).

The proportion of fermentable fiber is positively associated with the content of soluble fiber and negatively associated with the level of lignin. It is related with changes in the intestinal environment (pH, ammonia concentration, production of SCFAs). Both types of fiber, soluble low lignified (e.g., sugar beet pulp) and insoluble lignified (e.g., oats bran) affect swine intestinal health through different mechanisms: a) production of SCFA from hindgut fermentation, and b) improvement of intestinal motility and functionality (Nutritional requirements for swine, FEDNA, 2013)

Sows are well adapted to digest fiber. They are equipped with a more voluminous large intestine than piglets or fattening pigs. The digesta remains in the large intestine for 70-85% of the total digestion time, allowing it to be in contact with the hindgut microbiota. This particularity confers sows a much higher cellulolytic activity than young pigs for example. In fact, many of the bacteria able to digest fiber that are located in the rumen of a cow can also be identified in the colon of sows. It is important to note that cellulolytic bacteria need an anaerobic environment to proliferate and be metabolically active. However, this is not always the case due to the huge vascularization irrigating the intestinal mucosa that brings in oxygen and can have a negative impact on the microbial profile. The increase in fibrolytic bacteria population in the sows could be of interest also for the piglets, as it is well-demonstrated that the microbiota of the sows strongly influences the early colonization in the piglet.

Biliary acids emerge as a promising target for developing efficacious alternatives to the use of antibiotic as growth promoter. Indeed, it has been demonstrated that the use of antibiotics and zinc oxide at doses commonly used for stimulating growth or preventing post-weaning enteritis in pigs converge in promoting microbial production of bile acids (BA) in the intestine. This leads to tissue-specific modifications in the proportion of BA, thereby amplifying BA signaling in intestine, liver, and white adipose tissue. Activation of BA-regulated pathways ultimately reinforces the intestinal protection against bacterial infection and pathological secretion of fluids and electrolytes, attenuates inflammation in colon, alters protein and lipid metabolism in liver. Conceivably, these alterations could spare nutrients for growth and improve the metabolic efficiency of treated monogastric animals. Thus, promoting bacterial population able to produce these bile acids is of interest for promoting growth of animals.

The present disclosure follows from the unexpected finding that feeding monogastric animals with a *Saccharomyces cerevisiae* strain, or a composition comprising thereof impacts on feed efficiency of monogastric animals and on an average daily weight gain of monogastric animals by enhancing fiber digestibility of monogastric animals by influencing lower gut fermentation; and also by stimulating a bacterial population related to bile acids metabolism.

Without being bound to a particular theory, it is believed that part of the mode of action of *Saccharomyces cerevisiae* in the gut of monogastric animals has to do with the fast consumption of oxygen carried out by the yeast in both caecum and colon. This creates better anaerobic conditions where the anaerobic bacteria, comprising cellulolytic bacteria and bacteria involved in the bile acids metabolism can proliferate. As a result, more energy in the form of SCFA is released from the same diet and in a shorter time; while the change in the bile acids metabolism leads to better growth efficiency.

The present disclosure provides a composition comprising at least one biologically pure culture of *Saccharomyces cerevisiae* strain in amount effective for enhancing average daily gain of monogastric animals, and a suitable carrier.

The present disclosure further provides a composition comprising at least one biologically pure culture of *Saccharomyces cerevisiae* strain in amount effective for maintaining or growing a population of microorganisms in a gut of monogastric animals and/or a litter thereof, and a suitable carrier. In an embodiment, the population of microorganisms is either responsible of fiber digestion, of conversion of primary biliary acids into secondary biliary acids or both.

In an embodiment, the at least one biologically pure culture of *Saccharomyces cerevisiae* strain is a probiotic strain. In a further embodiment, the at least one biologically pure culture of *Saccharomyces cerevisiae* strain is a *Saccharomyces cerevisiae var. boulardii* strain. In yet a further embodiment, the at least one biologically pure culture of *Saccharomyces cerevisiae* strain is a *Saccharomyces cerevisiae var. boulardii* strain deposited under accession number 1-1079 at the CNCM (i.e. at the COLLECTION NATIONALE DE CULTURES DE MICROORGANISMES, Institut Pasteur, 25-28 rue du Docteur Roux, 75724 Paris Cedex 15, FRANCE).

The expression "in amount effective" when used herein will be understood to refer to an amount of at least one biologically pure culture of *Saccharomyces cerevisiae* strain which is at least sufficient to maintain or grow the population of microorganisms in a gut of monogastric animals and/or a litter thereof. The expression also refer to an amount of at least one biologically pure culture of *Saccharomyces cerevisiae* strain which is at least sufficient to enhance an average daily gain of monogastric animals.

In an embodiment, the amount of at least one biologically pure culture of *Saccharomyces cerevisiae* strain which is at least sufficient to maintain or grow the population of microorganisms in a gut of monogastric animals and/or a litter thereof; or to enhance an average daily gain of offspring from monogastric animals is an amount of at least 1x10⁷ CFU per kilogram of the composition, at least 1x10⁸ per kilogram of the composition or 1x10⁹ CFU per kilogram of the composition. In a further embodiment, the at least one biologically pure culture of *Saccharomyces cerevisiae* strain is in an amount ranging from 1x10⁷ CFU to 1x10¹² CFU per kilogram of the composition. In yet a further embodiment, the at least one biologically pure culture of *Saccharomyces cerevisiae* strain is in an amount ranging from 1x10⁸ CFU to 1x10¹² CFU per kilogram of the composition. In still a further embodiment, the at least one biologically pure culture of *Saccharomyces cerevisiae* strain is in an amount ranging from 1x10⁹ CFU to 1x10¹² CFU per kilogram of the composition.

In an embodiment, the suitable carrier is feed or food, more specifically any orally ingestible animal feed or food suitable for monogastric animals. The skilled person in the art will appreciate that feed or food may vary from one monogastric animals to another. The feed and the food including but not limited to a soup, pellets or a meal-based diet for swine and poultry, or kibbles, wet food and treats for dogs and cats. In a further embodiment, at least one biologically pure culture of *Saccharomyces cerevisiae* strain can be admixed with the basal diet of the monogastric animals. In an alternate embodiment, the at least one biologically pure culture of *Saccharomyces cerevisiae* strain can be top-dressed over feed or food. In a further alternate embodiment, the at least one biologically pure culture of *Saccharomyces cerevisiae* strain can be top-dressed over the basal diet of the monogastric animals.

In one embodiment, the monogastric animals are swine, dogs, cats, horses, rabbits or poultry. In a further embodiment the swine include sows, growing and fattening pigs and piglets. In yet a further embodiment the poultry include hens and chicks. In a furthermore embodiment, the monogastric animals are offspring thereof.

In an alternative embodiment, the compositions described above can further comprise at least one additional microorganism strain. The additional microorganism strain including but not limited to *Bacillus subtilis, B. amyloliquefaciens, B. licheniformis, Enterococcus faecium, Pediococcus acidilacti, Lactococcus lactis, Lactobacillus acidophilus, L. casei, L. plantarum, L. rhamnosus* or a mixture thereof.

In another alternative embodiment, the compositions comprising at least one biologically pure culture of *Saccharomyces cerevisiae* strain do not comprise any *Enterococcus* strain. The compositions may not comprise any bacterial strain and/or may not comprise any additional yeast strain. The compositions may not comprise any additional microorganism strain. The composition may consist or consist essentially of one or more biologically pure culture of *Saccharomyces cerevisiae* strain, and a suitable carrier.

The compositions in accordance with the present description can be in any suitable form to be served to the monogastric animals. The skilled person in the art would know what form is preferable for each monogastric animal species. For the sake of exemplifying the various form available, the compositions in accordance with the present disclosure can be in the form of a gelatin capsules, a pressed tablets, a gel caps, an animal feed or supplements, animal food or liquid beverages.

The expression "population of microorganisms responsible of fiber digestion" when used herein will be understood to refer generally to microorganisms that are able to process complex plant polysaccharides thanks to their capacity to synthesize cellulolytic and hemicellulolytic enzymes (often referred to as *Fibrolytic* bacteria). Polysaccharides are present in plant cellular cell walls in a compact fiber form where they are mainly composed of cellulose and hemicellulose.

The expression "population of microorganisms responsible of conversion of primary biliary acids into secondary biliary acids" when used herein will be understood to refer generally to microorganisms that are able to metabolize primary biliary acids. Biliary acids are produced from cholesterol and other acid steroids by the liver of mammals.

In an embodiment, the population of microorganisms responsible of fiber digestion is from the *Fibrobacteres* phylum, from the *Actinobacteria* phylum, or both. In a further embodiment the population of microorganisms responsible of fiber digestion is from the *Fibrobacter* genus, *Bifidobacteriaceae* family or both. In still a further embodiment, the population of microorganisms responsible of fiber digestion is a *Fibrobacter intestinalis,* a *Fibrobacter succinogenes,* or both.

In an embodiment, the microorganisms responsible of conversion of primary biliary acids into secondary biliary acids are from *Actinobacteria* phylum. In a further embodiment, the microorganisms responsible of conversion of primary biliary acids into secondary biliary acids are from *Coriobacteraceae* family. In yet a further embodiment, the microorganisms responsible of conversion of primary biliary acids into secondary biliary acids are from *Collinsella sp.,Olsenella sp.,* or both.

In either cases, the population of microorganisms also allows for enhancing an average daily gain of monogastric animals.

The present disclosure further provides for a feed or a food additive comprising a composition as defined in all the previous embodiments

The present disclosure further provides for a use of the composition described in all the embodiments above to enhance fiber digestibility of monogastric animals. In an embodiments of the use, the fiber digestibility of monogastric animals fed with the composition as defined in the different embodiments above enhanced fiber digestibility of monogastric animals by at least 1, 2, 3, 4, 5, 10, 15, 20, 25, 30, 35, 40, 45, 50, 55, 60, 65, 70, 75, 80, 85, 90, 95 or 100% when compared to the fiber digestibility of a monogastric animal not fed with the composition.

In another embodiment, the composition described in the above embodiments is used for maintaining or growing the population of microorganisms in a gut of monogastric animals and/or a litter thereof. In a further embodiment of the use, the population of microorganisms in a gut of monogastric animals fed with the composition is enhanced by at least 10, 25, 30, 35, 40, 45, 50, 55, 60, 70, 80, 85, 90, 100, 105, 110, 115, 120, 125, 130, 135, 140, 145, 145, 150, 155, 160, 165, 170, 175, 180, 185, 190, 200, 205, 210, 215, 220, 225, 230, 235, 240, 245, 250, 255, 260, 265, 270, 275, 280, 285, 290, 295 or 300% when compared to the microorganism population in a monogastric animal not fed with the composition. In a further embodiment of the use, the population of microorganisms is responsible of fiber digestion, of conversion of primary biliary acids into secondary biliary acid or both.

In yet a further embodiment, the composition described in all the embodiments above is used for enhancing an average daily gain of monogastric animals. In a further embodiment, the average daily gain of monogastric animals fed with the composition is enhanced of at least 1, 2, 3, 4, 5, 10, 15, 20, 25, 30, 35, 40, 45, 50, 55, 60, 65, 70, 75, 80, 85, 90, 95 or 100% when compared to the average daily gain of monogastric animals not fed with the composition.

The present disclosure further provides for a method of maintaining or growing the population of microorganisms in a gut of monogastric animals and/or a litter thereof, the method comprises feeding to the monogastric animals an effective amount of the composition as defined in the embodiments above. In an embodiment, the population of microorganisms allows for or is responsible of fiber digestion, of conversion of primary biliary acids into secondary biliary acids or both In another embodiment of the method, the growth of the population of microorganisms in the gut of monogastric animals is enhanced by at least 10, 15, 20, 25, 30, 35, 40, 45, 50, 55, 60, 70, 80, 85, 90, 100, 105, 110, 115, 120, 125, 130, 135, 140, 145, 145, 150, 155, 160, 165, 170, 175, 180, 185, 190, 200, 205, 210, 215, 220, 225, 230, 235, 240, 245, 250, 255, 260, 265, 270, 275, 280, 285, 290, 295 or 300% when compared to the microorganism population in a monogastric animal not fed with the composition. In another embodiment of the method, the composition can be admixed with the basal diet of the animal.

In a further embodiment of the method, the amount of at least one biologically pure culture of *Saccharomyces cerevisiae* strain is an amount of at least 1x10⁷ CFU per kilogram of the composition, at least 1x10⁸ per kilogram of the composition or 1x10⁹ CFU per kilogram of the composition. In a further embodiment, the at least one biologically pure culture of *Saccharomyces cerevisiae* strain is in an amount ranging from 1x10⁷ CFU to 1x10¹² CFU per kilogram of the composition. In yet a further embodiment, the at least one biologically pure culture of *Saccharomyces cerevisiae* strain is in an amount ranging from 1x10⁸ CFU to 1x10¹² CFU per kilogram of the composition. In still a further embodiment, the at least one biologically pure culture of *Saccharomyces cerevisiae* strain is in an amount ranging from 1x10⁹ CFU to 1x10¹² CFU per kilogram of the composition.

In an embodiment of the method, the step of feeding to the monogastric animals an effective amount of the composition is done on a daily basis. In yet a further embodiment of the method, the step of feeding to the monogastric animals an effective amount of the composition is done on a daily basis for a period of at least 10, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59 or 60 days.

In an embodiment of the method described above, the monogastric animals is in gestation, in lactation, weaning or growing.

In an embodiment of the method, the step of feeding to the monogastric animals an effective amount of the composition is done at least 5 days before whelping. It was found that feeding monogastric animal with the composition in accordance with the present description was also beneficial for the gut of offspring. In a further embodiment, the growth of the population of microorganisms in a gut of offspring from monogastric animals fed with the composition is enhanced when compared to the population of microorganisms responsible of fiber digestion in a gut of offspring from monogastric animals not fed with the composition. More specifically, the growth of the population of microorganisms responsible of fiber digestion in the gut of the offspring from monogastric animals fed with the composition is enhanced by at least 10, 15, 20, 25, 30, 35, 40, 45, 50, 55, 60, 70, 80, 85, 90, 100, 105, 110, 115, 120, 125, 130, 135, 140, 145, 145, 150, 155, 160, 165, 170, 175, 180, 185, 190, 200, 205, 210, 215, 220, 225, 230, 235, 240, 245, 250, 255, 260, 265, 270, 275, 280, 285, 290, 295 or 300% when compared to the microorganism population in the offspring from monogastric animals not fed with the composition.

In an alternate embodiment of the method described in the embodiment above, the step of feeding the composition to the monogastric animals can be done in combination with at least one antibiotic and/or Zinc Oxide. The antibiotics are those usually used by in agriculture for growing monogastric animals. The antibiotic used can be, for example, but not limited to is Amoxicillin, Doxycycline, Lyncomycin, colistin, Tiamulin, or any combinations thereof. Other suitable antibiotics can also be used in combination with the composition in accordance with the present disclosure in the method described above.

The present disclosure further provides for a method for enhancing an average daily gain of offspring from monogastric animals, the method comprising feeding to the monogastric animals an effective amount of the composition as described in all the embodiments above. In an embodiment of the method, the average daily gain of offspring from monogastric animals fed with an effective amount of composition in accordance with the present description is increased within a at least 21 days period compared to the average daily gain of offspring from monogastric animals not fed with an effective amount of the composition. In another embodiment of the method, the average daily gain of offspring from monogastric animals fed with the composition is enhanced of at least 1, 2, 3, 4, 5, 10, 15, 20, 25, 30, 35, 40, 45, 50, 55, 60, 65, 70, 75, 80, 85, 90, 95 or 100%when compared to the average daily gain of offspring from monogastric animals not fed with the composition.

In any of the uses and methods described above, the composition comprising at least one biologically pure culture of *Saccharomyces cerevisiae* strain may not comprise any *Enterococcus* strain. The composition may not comprise any bacterial strain and/or may not comprise any additional yeast strain. The composition may not comprise any additional microorganism strain. The methods and uses may not comprise administration of any *Enterococcus* strain. The methods and uses may not comprise administration of any additional bacterial strain and/or any additional yeast strain. The methods and uses may not comprise administration of any additional microorganism strain,

The uses described above may be non-therapeutic. The methods described above may be non-therapeutic. Also described herein is a composition as described above for use in a method for enhancing an average daily gain of monogastric animals, or for use in a method for maintaining or growing the population of micro-organisms in a gut of monogastric animals and/or a litter thereof. The method may be a method for treatment of the animal body by therapy.

In all the embodiments described above, the monogastric animals may be swine, dogs, cats, horses, rabbits, poultry or offspring thereof. The swine may be sows, growing and fattening pigs, and piglets.

The monogastric animals can be in gestation, in lactation, weaning or growing

### EXAMPLES:

Example 1 - Post-weaning piglets supplemented with *Saccharomyces cerevisiae var. boulardii* CNCM I-1079

### Materials and methods

Fecal samples were collected during a 50 day trial conducted on 288 piglets after weaning. Piglets were weaned at 21 day of age and followed a 3-phase feeding program. Basal diet was supplemented with antibiotics and ZnO in the first two phases (D0-D11 and D12-D33) and only with antibiotics in the third phase (D34-D50). Piglets were allocated to three groups: basal diet, basal diet supplemented with 2x 10⁹ CFU/kg *Saccharomyces cerevisiae var. boulardii* CNCM I-1079, diet without medication after D11 and with 2x 10⁹ CFU/kg *Saccharomyces cerevisiae var. boulardii* CNCM I-1079. Fecal samples were collected from

24 piglets per treatment group at D10, D34 and D50. Zootechnical performances were also measured: body weight (BW) was individually registered at the beginning and at the end of the trial and at each diet change. The feed intake (FI) was measured per pen at each change of diet.

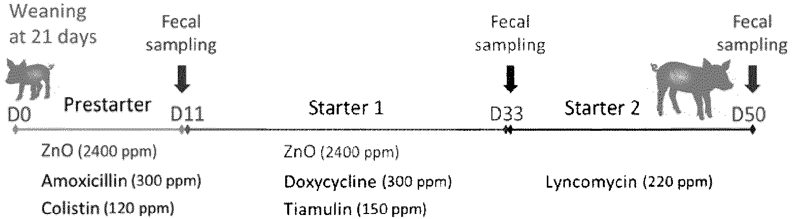

| **Treatment** | **Period (post-weaning)** | | |
|---|---|---|---|
| | **P1: 0-11** | **P2: 12-33** (3 weeks) | **P3: 34-50** (2,5 weeks) |
| **CTL+AB** | AB + ZnO | AB + ZnO | AB |
| **LSB+AB** | AB + ZnO + **LSB** | AB + ZnO + **LSB** | AB **+ LSB** |
| **LSB** | AB + ZnO + **LSB** | **LSB** | **LSB** |

### Microbial DNA extraction and 16S rRNA gene sequencing

Fecal samples collected were processed using a DNA extraction kit from Qiagen. Microbial DNA was extracted from 40-60 mg of feces using ZR-96 Soil Microbe DNA Kit^{™} (Zymo Research, Freiburg, Germany) according to the manufacturer's instruction. A 15 min bead beating step at 30 Hz was applied using a Retsch MM400 Mixer Mill. The V3 and V4 hypervariable regions of the 16S rRNA gene were amplified using the following primers:
**V3-V4 region**

| | |
|---|---|
| V3V4-343F | 5'- CTTTCCCTACACGACGCTCTTCCGATCTACGG**R**AGGCAGCAG -3' |
| V3V4-783R | 5'- GGAGTTCAGACGTGTGCTCTTCCGATCTTACCAGGGTATCTAATCCT -3' |

High-throughput sequencing was performed on a MiSeq sequencer using the Reagent Kit v3, according to the manufacturer's instruction (Illumina Inc., San Diego, CA).

### Bioinformatics analysis

Bioinformatics analyses were performed using GenoToul bioinformatics facility (Toulouse, France). Generated paired-end 250 bp sequences were assembled using Flash software (10bp minimum overlap, 10% maximum mismatch). Assembled sequences were processed using FROGS pipeline (Escudié et al., 2018). Briefly, sequences were clustered in Operational Taxonomic Units (OTUs) using SWARM algorithm (Mahé et al., 2014). Chimeric sequences were then detected by samples using UCHIME algorithm and removed from all samples (Edgar et al., 2011). A rarefaction step was applied to each sequencing dataset to avoid bias due to differences in sequencing depth. A filtering step was applied to remove all singletons (i.e. OTU represented by only one read). The generated OTU count table was normalized by total sum scaling. Taxonomic annotation of the OTUs was performed using the SILVA SSU database and BLAST+ and RDP algorithms. BLAST hits with identity and coverage alignments higher than 99% were kept for annotation. Otherwise, species were annotated as unknown and RDP classifier results were used for higher rank. Bootstrap thresholds were set to 0,9 and 0,8 respectively for annotation at the genus rank and higher ranks. Phylum, family and genus relative abundance tables were generated.

### Statistical analysis

*Zootechnical performances.* Average daily gain (ADG), feed intake (FI), and feed conversion ratio (FCR) were performed by a MIXED procedure of SAS (The SAS Stat. v.9.3) for repeated measurements on pen basis. The statistical model accounted for the main effects of treatment, sex, and time, also considering the interaction between treatment and time, treatment and sex, and treatment x time x sex. The experimental unit was the pen. Significance level was fixed for A,B P≤0.01 and a,b P≤0.05, while 0.05<P≤0.1 was considered as a trend.

*Microbiota analysis.* Microbiota statistical analyses were carried out using R software and RStudio software. A Kruskal-Wallis Rank Sum Test followed by Pairwise Test for Multiple Comparisons of Mean Rank Sums (Conover-Test, PMCMR R package) were used to analyze compositional data. Differential analyses were performed only on taxa represented by more than 0,005% of the total sequences. The Benjamini-Hochberg procedure was used to adjust generated P values. Contingency data were analyzed using a Fisher's exact test.

### Results

*Zootechnical performances.* Overall, there were significant differences (P<0.01) found in average daily gain (ADG), piglets fed T1 (yeast + antibiotic) being the ones growing faster than piglets fed Ctl (Ctl + antibiotic) and T2 (yeast alone) (Figure 1). There was an effect found in Feed Conversion Ratio (FCR), with piglets fed T1 converting the feed into body weight gain better than piglets fed Ctl (Figure 1). No significant differences were found for the feed intake, all piglets ate the same quantity of feed whatever the treatments.

*Microbiota analysis.* The relative abundance of the phyla *Fibrobacteres* increased with time (P<0,001) whatever the treatment was, probably due to the increase of fiber quantity reaching the colon.

The supplementation with yeast resulted in an increase of *Fibrobacteres* mean abundance (expressed as %) and in *Fibrobacteres* detection frequency (in %, P<0,05; Figure 2). This means that when piglets were supplemented with *Saccharomyces cerevisiae var. boulardii* CNCM I-1079, the abundance of Fibrobacteres in their colon was greater and that the presence of *Fibrobacteres* was more frequently detected in the colon of these piglets than in the control piglets.

The relative abundance of the phylum *Actinobacteria* and of the family of *Coriobacteriaceae* was also found to be increased in feces from piglets fed with *Saccharomyces cerevisiae var. boulardii* CNCM I-1079 on day 10 and on day 50 (Figure 3). The family *Coriobacteriaceae* was the most dominant family, accounting for more than 80%, among the Actinobacteria phylum. Among the *Coriobacteriaceae,* two genera, *Olsenella* and *Collinsella* increased on D10 and on D50 in piglets fed with *Saccharomyces cerevisiae var. boulardii* CNCM I-1079 (Figure 3).

### Example 2 - Sows supplemented with Saccharomyces cerevisiae var. boulardii CNCM I-1079 during gestation and lactation

### Materials and methods

### Experimental design

Fecal samples were collected during a trial conducted on sows (from 4 weeks before farrowing until weaning) and associated post-weaning piglets. Sows were fed a diet supplemented or not with 10⁹ CFU/kg *Saccharomyces cerevisiae var. boulardii* CNCM I-1079 (**Ctl** or **LSB** diet respectively). Piglets were weaned at 21 day of age and followed a 2-phase feeding program. Piglets born from control or *Saccharomyces cerevisiae var. boulardii* CNCM I-1079-treated sows were subsequently allocated to three groups: basal diet (**Ctl**), basal diet supplemented with 2500ppm ZnO, 420 ppm of antibiotics (Antibiotic group; **AB**), basal diet supplemented with 2x10⁹ CFU/kg *Saccharomyces cerevisiae var. boulardii* CNCM I-1079 (**SB**). Fecal samples were collected from 13 and 22 sows per treatment, respectively for primiparous and parity 2 sows. The same sows were sampled 2 days after move to farrowing house, and one day after farrowing. Fecal samples were collected from 10 piglets per treatment group, so 60 piglets in total. Piglets were sampled on weaning day, at day 6 and day 20 post-weaning.

### Microbial DNA extraction and 16S rRNA gene sequencing

Fecal samples collected were processed as followed: microbial DNA was extracted from 40-60 mg of feces using ZR-96 Soil Microbe DNA Kit^{™} (Zymo Research, Freiburg, Germany) according to the manufacturer's instruction. A 15 min bead beating step at 30 Hz was applied using a Retsch MM400 Mixer Mill. The V4 and V5 hypervariable regions of the 16S rRNA gene were amplified using the following primers:
**V4-V5 region**

| | |
|---|---|
| V4V5-515F | 5'- CTTTCCCTACACGACGCTCTTCCGATCTGTG**Y**CAGC**M**GCCGCGGTA -3' |
| V4V5-928R | 5'- GGAGTTCAGACGTGTGCTCTTCCGATCTCCCCG**Y**CAATTC**M**TTT**R**AGT -3' |

High-throughput sequencing was performed on a MiSeq sequencer using the Reagent Kit v3, according to the manufacturer's instruction (Illumina Inc., San Diego, CA).

### Bioinformatics analyses

Bioinformatics analyses were performed using GenoToul bioinformatics facility (Toulouse, France). Generated paired-end 250 bp sequences were assembled using Flash software (10bp minimum overlap, 10% maximum mismatch). Assembled sequences were processed using FROGS pipeline (Escudié et al., 2018). Briefly, sequences were clustered in Operational Taxonomic Units (OTUs) using SWARM algorithm (Mahé et al., 2014). Chimeric sequences were then detected by samples using UCHIME algorithm and removed from all samples (Edgar et al., 2011). A rarefaction step was applied to each sequencing dataset to avoid bias due to differences in sequencing depth. A filtering step was applied to remove all singletons (i.e. OTU represented by only one read). The generated OTU count table was normalized by total sum scaling. Taxonomic annotation of the OTUs was performed using the SILVA SSU database and BLAST+ and RDP algorithms. BLAST hits with identity and coverage alignments higher than 99% were kept for annotation. Otherwise, species were annotated as unknown and RDP classifier results were used for higher rank. Bootstrap thresholds were set to 0,9 and 0,8 respectively for annotation at the genus rank and higher ranks. Phylum, family and genus relative abundance tables were generated.

### Statistical analyses

*Zootechnical performances.* Average daily gain (ADG), feed intake (FI), and feed conversion ratio (FCR) were performed by a MIXED procedure of SAS (The SAS Stat. v.9.3) for repeated measurements. The statistical model accounted for the main effects of treatment of the sow, treatment of the piglet, and time, also considering the interactions. Significance level was fixed for A,B P≤0.01 and a,b P≤0.05, while 0.05<P≤0.1 was considered as a trend.

*Microbiota statistical analyses.* They were carried out using R software and RStudio software. A Kruskal-Wallis Rank Sum Test followed by Pairwise Test for Multiple Comparisons of Mean Rank Sums (Conover-Test, PMCMR R package) were used to analyze compositional data. Differential analyses were performed only on taxa represented by more than 0,005% of the total sequences. The Benjamini-Hochberg procedure was used to adjust generated P values. Contingency data were analyzed using a Fisher's exact test.

### Results

*Zootechnical performance.* A supplementation of sows with *Saccharomyces cerevisiae var. boulardii* CNCM I-1079 resulted in a significant increase in body weight of the piglets at weaning (Tableau 1). Furthermore, an effect of the supplementation of the sows is showed on growth performance on post-weaning performance, up to 35 days of age (Table 2).

These results demonstrates that when the sows were fed with *Saccharomyces cerevisiae var. boulardii* CNCM I-1079, the growth of their piglets was stronger than the growth obtained in the piglets which mothers were not fed with *Saccharomyces cerevisiae var. boulardii* CNCM I-1079.

**Table 1- Effect of a supplementation with Saccharomyces cerevisiae var. boulardii CNCM I-1079 (LSB) of the sow diet on performance at weaning. ADFI: Average Daily Feed Intake**

| | control | LSB | Standard Error | P value |
|---|---|---|---|---|
| ADFI (kg) | 4.2 | 4.3 | 0.1 | 0.63 |
| Average weaning weight (kg) | 5.9 | 6.3 | 0.1 | 0.03 |
| Mortality (%) (LS means) | 2.8 | 1.9 | 0.01 | 0.22 |

**Table 2 - Effect of a supplementation with Saccharomyces cerevisiae var. boulardii CNCM I-1079 (LSB) of the sow and the post-weaning diets on growth performance of piglets; ADG: Average Daily Gain; ADFI: Average Daily Feed Intake; FCR: Feed Conversion ratio.**

| *Dependent Variable* | *LSB_SOW* | *Mean* | *Std. Error* | |
|---|---|---|---|---|
| *ADG 0-35* | *NO* | *311,346* | *9,426* | ***0.014*** |
| | *YES* | *346,488* | *9,426* | |
| *ADFI* | *NO* | *416,791* | *11,796* | ***0.017*** |
| | *YES* | *458,931* | *11,796* | |
| *FCR* | *NO* | *1,343* | *0,027* | *0.741* |
| | *YES* | *1,33* | *0,027* | |

*Microbiota analysis.* In sows, we evidenced an effect of *Saccharomyces cerevisiae var. boulardii* CNCM I-1079 supplementation on the microbiota alpha-diversity. A higher homogeneity of the samples was observed within *Saccharomyces cerevisiae var. boulardii* CNCM I-1079 group.

*Saccharomyces cerevisiae var. boulardii* CNCM I-1079 supplementation in sows was associated with a significant higher relative abundance of the phylum of *Fibrobacteres* before farrowing (Figure 4).

In addition, we observed a higher frequency of *Fibrobacter intestinalis* in piglets whose mothers were fed with *Saccharomyces cerevisiae var. boulardii* CNCM I-1079 supplementation (Figure 5). Strikingly, those mother effects were persistent up to 20 days post weaning whatever the post-weaning diets. Furthermore, medication in piglets decreased *Fibrobacter intestinalis* frequency; while, interestingly, *Saccharomyces cerevisiae var. boulardii* CNCM I-1079 supplementation in sows delayed the effect of medication in piglets (Figure 5). Such results suggest a maternal imprinting effect when sows are fed with *Saccharomyces cerevisiae var. boulardii* CNCM I-1079, probably by impacting early microbiota colonization of their piglets.

A positive correlation between Fibrobacteres relative abundance in sows before farrowing and piglets performance (average weaning weight) was also seen, as shown in Figure 6.

All these results taken together suggest that the supplementation with *Saccharomyces cerevisiae var. boulardii* CNCM I-1079 led to an increase in the population of *Fibrobacteres* in the colon of the sows, but also in the colon of the piglets which sows were fed with *Saccharomyces cerevisiae var. boulardii* CNCM I-1079. In the first example, in addition to the effect on *Fibrobacteres,* we observed an effect on *Actinobacteria* phylum, and especially on *Collinsella* and *Olsenella,* 2 genera described to be able to metabolize primary bile acids. In the 2 provided examples, the piglets, at the same time, exhibited greater growth.

While the invention has been described in connection with specific embodiments thereof, it will be understood that the scope of the claims should not be limited by the preferred embodiments set forth in the examples, but should be given the broadest interpretation consistent with the description as a whole.

Further embodiments of the invention:
1. A composition comprising at least one biologically pure culture of *Saccharomyces cerevisiae* strain in amount effective for enhancing an average daily gain of monogastric animals, and a suitable carrier.
2. A composition comprising at least one biologically pure culture of *Saccharomyces cerevisiae* strain in amount effective for maintaining or growing a population of microorganisms in a gut of monogastric animals and/or a litter thereof, and a suitable carrier.
3. The composition of embodiment 2, wherein:
   (a) the population of microorganisms is responsible of fiber digestion, of conversion of primary biliary acids into secondary biliary acids or both, optionally wherein the microorganisms responsible of fiber digestion are from *Fibrobacteres* phylum, *Actinobacteria* phylum or both, preferably wherein the microorganisms responsible of fiber digestion are from *Fibrobacteres* genus, *Bifidobacteriaceae* family or both, more preferably wherein the microorganisms responsible of fiber digestion is a *Fibrobacter intestinalis* strain; or
   (b) the microorganisms responsible of conversion of primary biliary acids into secondary biliary acids are from *Actinobacteria* phylum, optionally wherein the microorganisms responsible of conversion of primary biliary acids into secondary biliary acids are from *Coriobacteraceae* family.
4. The composition of embodiment 2 or 3, wherein the population of microorganisms allows for enhancing an average daily gain of monogastric animals.
5. The composition of any one of embodiment 1 to 4, wherein:
   (a) the *Saccharomyces cerevisiae* strain is a probiotic strain;
   (b) the *Saccharomyces cerevisiae* strain is a *Saccharomyces cerevisiae var boulardii* strain;
   (c) the *Saccharomyces cerevisiae* strain is a *Saccharomyces cerevisiae var boulardii* strain deposited under accession number 1-1079 at the CNCM;
   (d) the suitable carrier is feed;
   (e) the at least one biologically pure culture of *Saccharomyces cerevisiae* strain is in an amount of at least 1 x 10⁹ CFU per kilogram of the composition;
   (f) the composition further comprises at least one additional microorganism strain;
   (g) the composition is in the form of gelatin capsules, pressed tablets, gel caps, an animal feed or liquid beverages; and/or
   (h) the monogastric animals are swines, dogs, cats, horses, rabbits, or poultry or offspring thereof.
6. A feed or food additive comprising the composition as defined in any one of embodiments 1 to 5.
7. Use of the composition as defined in any one of embodiments 1, 4 and 5, for enhancing an average daily gain of monogastric animals.
8. The use of embodiment 7, wherein the average daily gain of monogastric animals fed with the composition is enhanced of at least 1, 2, 3, 4, 5, 10, 15, 20, 25, 30, 35, 40, 45, 50, 55, 60, 65, 70, 75, 80, 85, 90, 95 or 100% when compared to the average daily gain of monogastric animals not fed with the composition.
9. Use of the composition as defined in any one of embodiments 2 to 5, for maintaining or growing the population of microorganisms in a gut of monogastric animals and/or a litter thereof.
10. The use of embodiment 9, wherein:
   (a) the population of microorganisms in a gut of monogastric animals fed with the composition is enhanced by at least 10, 15, 20 25, 30, 35, 40, 45, 50, 55, 60, 70, 80, 85, 90, 100, 105, 110, 115, 120, 125, 130, 135, 140, 145, 145, 150, 155, 160, 165, 170, 175, 180, 185, 190, 200, 205, 210, 215, 220, 225, 230, 235, 240, 245, 250, 255, 260, 265, 270, 275, 280, 285, 290, 295 or 300% when compared to the microorganism population of a monogastric animal not fed with the composition; and/or
   (b) the population of microorganisms is responsible of fiber digestion, of conversion of primary biliary acids into secondary biliary acid or both.
11. A method of maintaining or growing the population of microorganisms in a gut of monogastric animals and/or a litter thereof, the method comprising feeding to the monogastric animals an effective amount of the composition according to any one of embodiments 2 to 5.
12. The method of embodiment 11, wherein:
   (a) the growth of the population of in the gut of monogastric animals is enhanced by at least 10, 15, 20, 25, 30, 35, 40, 45, 50, 55, 60, 70, 80, 85, 90, 100, 105, 110, 115, 120, 125, 130, 135, 140, 145, 145, 150, 155, 160, 165, 170, 175, 180, 185, 190, 200, 205, 210, 215, 220, 225, 230, 235, 240, 245, 250, 255, 260, 265, 270, 275, 280, 285, 290, 295 or 300% when compared to the microorganism population of a monogastric animal not fed with the composition;
   (b) the method comprise admixing the composition with the basal diet of the animal;
   (c) the step of feeding to the monogastric animals an effective amount of the composition is done on a daily basis for a period of at least 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59 or 60 days;
   (d) the composition fed to the monogastric animals further comprises at least one antibiotic, Zinc Oxide or a mixture thereof, optionally wherein the antibiotic is Amoxicillin, Doxycycline, Lyncomycin, colistin, Tiamulin, or any combination thereof;
   (e) the monogastric animals is in gestation, in lactation, weaning or growing; and/or
   (f) the growth of the population of microorganisms in a gut of offspring from monogastric animals fed with the composition is enhanced when compared to the population of microorganisms in a gut of offspring from monogastric animals not fed with the composition, optionally wherein the growth of the population of microorganisms in the gut of the offspring from monogastric animals fed with the composition is enhanced of at least 2, 3, 4, 5, 10, 15, 20, 25, 30, 35, 40, 45, 50, 55, 60, 65, 70, 75, 80, 85, 90, 95 or 100% when compared to the microorganism population in the offspring from monogastric animals not fed with the composition.
13. A method for enhancing an average daily gain of monogastric animals, the method comprising feeding to the monogastric animals an effective amount of the composition according to any one of embodiments 1, 4 and 5.
14. The method of embodiment 13, wherein:
   (a) the average daily gain of monogastric animals fed an effective amount of the composition is increased within at least 21 days period compared to the average daily gain of monogastric animals not fed with an effective amount of the composition;
   (b) the average daily gain of monogastric animals fed with the composition is enhanced of at least 1, 2, 3, 4, 5, 10, 15, 20, 25, 30, 35, 40, 45, 50, 55, 60, 65, 70, 75, 80, 85, 90, 95 or 100% when compared to the average daily gain of monogastric animals not fed with the composition;
   (c) the monogastric animals are swine, dogs, cats, horses, rabbits, poultry or offspring thereof; optionally wherein the swine is selected from the group consisting of sows, growing and fattening pigs, and piglets; and/or
   (d) the monogastric animals is in gestation, in lactation, weaning or growing

The following further embodiments are disclosed:
1. A composition comprising at least one biologically pure culture of *Saccharomyces cerevisiae* strain in amount effective for enhancing an average daily gain of monogastric animals, and a suitable carrier.
2. A composition comprising at least one biologically pure culture of *Saccharomyces cerevisiae* strain in amount effective for maintaining or growing a population of microorganisms in a gut of monogastric animals and/or a litter thereof, and a suitable carrier.
3. The composition of embodiment 2, wherein the population of microorganisms is responsible of fiber digestion, of conversion of primary biliary acids into secondary biliary acids or both.
4. The composition of embodiment 3, wherein the microorganisms responsible of fiber digestion are from *Fibrobacteres* phylum, *Actinobacteria* phylum or both
5. The composition of embodiment 4, wherein the microorganisms responsible of fiber digestion are from *Fibrobacteres* genus, *Bifidobacteriaceae* family or both.
6. The composition of embodiment 5, wherein the microorganisms responsible of fiber digestion is a *Fibrobacter intestinalis* strain.
7. The composition of embodiment 2, wherein the microorganisms responsible of conversion of primary biliary acids into secondary biliary acids are from *Actinobacteria* phylum.
8. The composition of embodiment 7, wherein the microorganisms responsible of conversion of primary biliary acids into secondary biliary acids are from *Coriobacteraceae* family.
9. The composition of any one of embodiments 2 to 8, wherein the population of microorganisms allows for enhancing an average daily gain of monogastric animals.
10. The composition of any one of embodiments 1 to 9, wherein the *Saccharomyces cerevisiae* strain is a probiotic strain.
11. The composition of any one of embodiments 1 to 10, wherein the *Saccharomyces cerevisiae* strain is a *Saccharomyces cerevisiae var boulardii* strain.
12. The composition of any one of embodiments 1 to 11, wherein the *Saccharomyces cerevisiae* strain is a *Saccharomyces cerevisiae var boulardii* strain deposited under accession number 1-1079 at the CNCM.
13. The composition of any one of embodiments 1 to 12, wherein the suitable carrier is feed.
14. The composition of any one of embodiments 1 to 13, wherein the at least one biologically pure culture of Saccharomyces cerevisiae strain is in an amount of at least 1x10⁹ CFU per kilogram of the composition.
15. The composition of any one of embodiments 1 to 14, further comprising at least one additional microorganism strain.
16. The composition of any one of embodiments 1 to 15, wherein the composition is in the form of gelatin capsules, pressed tablets, gel caps, an animal feed or liquid beverages.
17. The composition of any one of embodiments 1 to 16, wherein the monogastric animals are swines, dogs, cats, horses, rabbits, or poultry or offspring thereof.
18. A feed or food additive comprising the composition as defined in any one of embodiments 1 to 17.
19. Use of the composition as defined in any one of embodiments 1 and 9 to 17, for enhancing an average daily gain of monogastric animals.
20. The use of embodiment 19, wherein the average daily gain of monogastric animals fed with the composition is enhanced of at least 1, 2, 3, 4, 5, 10, 15, 20, 25, 30, 35, 40, 45, 50, 55, 60, 65, 70, 75, 80, 85, 90, 95 or 100% when compared to the average daily gain of monogastric animals not fed with the composition.
21. Use of the composition as defined in any one of embodiments 2 to 17, for maintaining or growing the population of microorganisms in a gut of monogastric animals and/or a litter thereof.
22. The use of embodiment 21, wherein the population of microorganisms in a gut of monogastric animals fed with the composition is enhanced by at least 10, 15, 20 25, 30, 35, 40, 45, 50, 55, 60, 70, 80, 85, 90, 100, 105, 110, 115, 120, 125, 130, 135, 140, 145, 145, 150, 155, 160, 165, 170, 175, 180, 185, 190, 200, 205, 210, 215, 220, 225, 230, 235, 240, 245, 250, 255, 260, 265, 270, 275, 280, 285, 290, 295 or 300% when compared to the microorganism population of a monogastric animal not fed with the composition.
23. The use of embodiment 21 or 23, wherein the population of microorganisms is responsible of fiber digestion, of conversion of primary biliary acids into secondary biliary acid or both.
24. A method of maintaining or growing the population of microorganisms in a gut of monogastric animals and/or a litter thereof, the method comprising feeding to the monogastric animals an effective amount of the composition according to any one of embodiments 2 to 17.
25. The method of embodiment 24, wherein the growth of the population of in the gut of monogastric animals is enhanced by at least 10, 15, 20, 25, 30, 35, 40, 45, 50, 55, 60, 70, 80, 85, 90, 100, 105, 110, 115, 120, 125, 130, 135, 140, 145, 145, 150, 155, 160, 165, 170, 175, 180, 185, 190, 200, 205, 210, 215, 220, 225, 230, 235, 240, 245, 250, 255, 260, 265, 270, 275, 280, 285, 290, 295 or 300% when compared to the microorganism population of a monogastric animal not fed with the composition.
26. The method of embodiment 24 or 25, wherein the method comprise admixing the composition with the basal diet of the animal.
27. The method of any one of embodiments 24 to 26, wherein the step of feeding to the monogastric animals an effective amount of the composition is done on a daily basis for a period of at least 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59 or 60 days.
28. The method of any one embodiments 24 to 27, wherein the composition fed to the monogastric animals further comprises at least one antibiotic, Zinc Oxide or a mixture thereof.
29. The method of embodiment 28, wherein the antibiotic is Amoxicillin, Doxycycline, Lyncomycin, colistin, Tiamulin, or any combinations thereof.
30. The method of any one of embodiments 24 to 29, wherein the monogastric animals is in gestation, in lactation, weaning or growing.
31. The method of any one of embodiments 24 to 30, wherein the growth of the population of microorganisms in a gut of offspring from monogastric animals fed with the composition is enhanced when compared to the population of microorganisms in a gut of offspring from monogastric animals not fed with the composition.
32. The method of embodiment 31, wherein the growth of the population of microorganisms in the gut of the offspring from monogastric animals fed with the composition is enhanced of at least 2, 3, 4, 5, 10, 15, 20, 25, 30, 35, 40, 45, 50, 55, 60, 65, 70, 75, 80, 85, 90, 95 or 100% when compared to the microorganism population in the offspring from monogastric animals not fed with the composition.
33. A method for enhancing an average daily gain of monogastric animals, the method comprising feeding to the monogastric animals an effective amount of the composition according to any one of embodiments 1 and 9 to 17.
34. The method of embodiment 33, wherein the average daily gain of monogastric animals fed an effective amount of the composition is increased within at least 21 days period compared to the average daily gain of monogastric animals not fed with an effective amount of the composition.
35. The method of embodiment 33 or 34, wherein the average daily gain of monogastric animals fed with the composition is enhanced of at least 1, 2, 3, 4, 5, 10, 15, 20, 25, 30, 35, 40, 45, 50, 55, 60, 65, 70, 75, 80, 85, 90, 95 or 100% when compared to the average daily gain of monogastric animals not fed with the composition.
36. The method of any one of embodiments 33 to 35, wherein the monogastric animals are swine, dogs, cats, horses, rabbits, poultry or offspring thereof.
37. The method of embodiment 36, wherein the swine is selected from the group consisting of sows, growing and fattening pigs, and piglets.
38. The method of any one of embodiments 33 to 36, wherein the monogastric animals is in gestation, in lactation, weaning or growing.
39. The composition, method or use of any one of embodiments 1-14 and 16-38, wherein the composition does not comprise any *Enterococcus* strain.
40. The composition, method or use of embodiment 39, wherein the composition does not comprise any bacterial strain and/or any additional yeast strain, or does not comprise any additional microorganism strain.

## Claims

1. A non-therapeutic use of a composition for enhancing the average daily gain or increasing the average weaning weight of offspring of a sow,
wherein the composition comprises at least one biologically pure culture of *Saccharomyces cerevisiae var boulardii* strain deposited under accession number 1-1079 at the CNCM in an amount effective for enhancing average daily gain, and a suitable carrier,
wherein the composition does not comprise any *Enterococcus* strain, and
wherein the use comprises feeding the composition to the sow during gestation and/or lactation.

2. The use of claim 1, wherein the average daily gain of the offspring from the sow fed with the composition is increased within an at least 21 days period compared to the average daily gain of offspring from a sow not fed with an effective amount of the composition.

3. The use of claim 1 or 2, wherein the average daily gain of the offspring from the sow fed with the composition is enhanced of at least 1, 2, 3, 4, 5, 10, 15, 20, 25, 30, 35, 40, 45, 50, 55, 60, 65, 70, 75, 80, 85, 90, 95 or 100% when compared to the average daily gain of offspring from a sow not fed with the composition.

4. The use of any one of claims 1-3, wherein the amount of the at least one biologically pure culture of *Saccharomyces cerevisiae* strain which is at least sufficient to enhance the average daily gain of offspring from the sow is an amount of at least 1x10⁷ CFU per kilogram of the composition, at least 1x10⁸ per kilogram of the composition or at least 1x10⁹ CFU per kilogram of the composition.

5. The use of any one of claims 1-4, wherein the at least one biologically pure culture of *Saccharomyces cerevisiae* strain is in an amount ranging from 1x10⁷ CFU to 1x10¹² CFU per kilogram of the composition.

6. The use of any one of claims 1-5, wherein the amount of the at least one biologically pure culture of *Saccharomyces cerevisiae* strain is 1x10⁹ CFU per kilogram of the composition.

7. The use of any one of claims 1-6, wherein the average daily gain of the offspring from the sow fed with the composition is enhanced up to 35 days of age.

8. The use of any one of claims 1-7, wherein the *Saccharomyces cerevisiae* strain has been admixed with the basal diet of the sow.

9. The use of any one of claims 1-8, wherein:
(a) the suitable carrier is feed; and/or
(b) the composition is in the form of a gelatin capsule, a pressed tablet, a gel cap, an animal feed or supplement, or a liquid beverage.

10. The use of any one of claims 1-9, wherein the composition further comprises at least one additional microorganism strain.

11. The use of any one of claims 1-10, wherein the composition does not comprise any bacterial strain and/or any additional yeast strain.

12. The use of any one of claims 1-9, wherein the composition does not comprise any additional microorganism strain.
